# EUROPEAN PATENT APPLICATION

(11) **EP 3 831 932 A1**
(43) Date of publication of application: **09.06.2021**
(21) Application number: 19841526.7
(22) Date of filing: 26.07.2019
(51) Int. Cl.: C12N 5/02, C12N 1/00, C12N 5/0735, C12N 5/074, C12N 5/10

(54) **SUSPENSION CULTURING ADDITIVE, SUSPENSION CULTURING MEDIUM AND SUSPENSION CULTURING METHOD FOR ANIMAL CELLS**

(30) Priority: 27.07.2018 JP 2018141909
(71) Applicant: AJINOMOTO CO., INC., Chuo-ku Tokyo 104-8315 (JP)
(72) Inventor: ITO, Kenichiro, Kawasaki-shi, Kanagawa 210-8681 (JP); FUROMITSU, Shumpei, Kawasaki-shi, Kanagawa 210-8681 (JP); OHYA, Yusuke, Kawasaki-shi, Kanagawa 210-8681 (JP); HIGUCHI, Takuya, Kawasaki-shi, Kanagawa 210-8681 (JP)
(74) Representative: Hoffmann Eitle
(86) International application number: PCT/JP2019/029535
(87) International publication number: WO 2020/022511

(57) **Abstract**

The present invention relates to an additive and a medium containing water-soluble polymer for suspension culture of animal cells, as well as a method for suspension culture animal cell including suspension culture of animal cells in a medium containing a water-soluble polymer. The present invention can provide an additive for suspension culture, a medium for suspension culture, and a method for suspension culture of animal cells that suppress precipitation of medium components such as insulin and the like due to physical stimulation of stirring, shaking, circulation, gas bubbling, or the like in suspension culture of animal cells, and can improve the culture efficiency of animal cells and the quality of cultured cells.

## Description

### [Technical Field]

The present invention relates to an additive for suspension culture, a medium for suspension culture, and a method for suspension culture of animal cells.

### [Background Art]

Many animal cells including stem cells such as embryonic stem cells, and induced pluripotent stem cells have been proliferated and maintained by adhesion culture using human-type recombinant matrix such as Matrigel, vitronectin and laminin as scaffold materials.

However, to apply animal cells to research, substance production, medical treatment, and the like, a culture method for efficiently proliferating them is required. As a method for culturing a large amount of animal cells, a method of suspension culture by stirring with an impeller, a method of culturing by circulating the medium by using a peristaltic pump, a method of culturing while performing gas bubbling from the bottom surface by using a sparger, and the like have been widely used.

In the culture of many animal cells, a serum-free medium containing no serum that may contain unidentified factors, prions, viruses, and the like, or a low albumin medium having a low albumin content is used. However, such medium is used for the above-mentioned suspension culture with stirring, etc., precipitation has been reported to occur in the medium. It is considered that insulin added as a factor necessary for cell growth to a serum-free medium or a low albumin medium precipitated by physical stimulation such as stirring, circulation, gas bubbling, or the like (non-patent document 1).

The precipitation of medium components decreases cell proliferation. To perform efficient culture of animal cells, therefore, it is desirable to suppress such precipitation.

### [Document List]

### [Non-patent document]

Non-patent document 1: D. Massai et al., Sci. Rep. 7 3950 (2017)

### [Summary of Invention]

### [Technical Problem]

The present invention has been made given the above-mentioned situation. The present inventors have confirmed by Matrix Assisted Laser Desorption/Ionization-Time of Flight Mass Spectrometry (MALDI-TOFMS) that the precipitate produced by physical stimulation in suspension culture using a serum-free medium or a low albumin medium is insulin.

Therefore, an object of the present invention is to provide an additive for suspension culture, a medium for suspension culture, and a method for suspension culture of animal cells that suppress precipitation of medium components such as insulin and the like due to physical stimulation of stirring, shaking, circulation, gas bubbling, or the like in suspension culture of animal cells, and can improve the culture efficiency of animal cells and the quality of cultured cells.

### [Solution to Problem]

The present inventors have conducted intensive studies in an attempt to solve the above-mentioned problems, and found that the precipitation of medium components such as insulin and the like caused by physical stimulation can be favorably suppressed by adding a water-soluble polymer to a medium containing insulin and the like for suspension culture of animal cells, which resulted in the completion of the present invention.

That is, the present invention relates to the following.
[1] An additive for suspension culture of an animal cell, comprising a water-soluble polymer.
[2] The additive of [1], wherein the water-soluble polymer is a non-ionic water-soluble polymer having surface activity.
[3] The additive of [2], wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.
[4] The additive of any of [1] to [3], wherein the animal cell is a stem cell.
[5] The additive of [4], wherein the stem cell is one kind or two or more kinds selected from the group consisting of an adult stem cell, an embryonic stem cell, and an induced pluripotent stem cell.
[6] The additive of any of [1] to [5], wherein the additive is for suppressing precipitation of a medium component.
[7] The additive of [6], wherein the medium component is insulin.
[8] A medium for suspension culture of an animal cell, comprising a water-soluble polymer.
[9] The medium of [8], wherein the water-soluble polymer is a non-ionic water-soluble polymer having surface activity.
[10] The medium of [9], wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.
[11] The medium of any of [8] to [10], wherein the medium is for suspension culture of a stem cell.
[12] The medium of [11], wherein the stem cell is one kind or two or more kinds selected from the group consisting of an adult stem cell, an embryonic stem cell and an induced pluripotent stem cell.
[13] The medium of any of [8] to [12], wherein precipitation of a medium component is suppressed.
[14] The medium of [13], wherein the medium component is insulin.
[15] A method for suspension culture of an animal cell, comprising suspension culturing the animal cell in a medium comprising a water-soluble polymer.
[16] The method of [15], wherein the water-soluble polymer is a non-ionic water-soluble polymer having surface activity.
[17] The method of [16], wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.
[18] The method of any of [15] to [17], wherein the animal cell is a stem cell.
[19] The method of [18], wherein the stem cell is one kind or two or more kinds selected from the group consisting of an adult stem cell, an embryonic stem cell and an induced pluripotent stem cell.
[20] The method of any of [15] to [19], wherein the suspension culturing comprises stirring, shaking, circulation, or gas bubbling.
[21] The method of any of [15] to [20], wherein the animal cell is suspension cultured in a medium in which precipitation of a medium component is suppressed.
[22] The method of [21], wherein the medium component is insulin.
[23] The method of any of [15] to [22], wherein the animal cell is suspension cultured by forming a cell aggregate.

### [Advantageous Effects of Invention]

The present invention can provide an additive capable of favorably suppressing precipitation of a medium component such as insulin or the like, which is produced by physical stimulation such as stirring, shaking, circulation, gas bubbling or the like, by adding same to a medium containing insulin and the like for suspension culture of animal cells.

The present invention can also provide a medium containing insulin and the like for suspension culture of animal cells, in which precipitation of medium components such as insulin and the like is suppressed well even when physical stimulation such as stirring, shaking, circulation, gas bubbling or the like is applied, and suspension culture of animal cells can be performed while performing stirring, shaking, circulation, gas bubbling and the like in a medium containing insulin and the like for suspension culture of animal cells.

As a result, cell aggregates with controlled size can be efficiently formed, and the culture efficiency of animal cells and the quality of cultured cells can be improved.

In particular, precipitation of medium components is suppressed during both maintenance culture of undifferentiated cells such as stem cell and the like in a maintenance medium and differentiation induction thereof in a differentiation induction medium, and both the undifferentiated state maintenance rate during maintenance culture and the differentiation rate during differentiation induction can be improved.

### [Brief Description of Drawings]

Fig. 1 shows the influence of each addition concentration of poly(vinyl alcohol) (PVA) and poloxamer (Kolliphor P188 BIO) on the precipitation suppressive effect of insulin in Example 2. The bar in the Figure indicates 500 µm.
Fig. 2 shows the influence of the addition concentration of poloxamer (Kolliphor P188 BIO) on the cell proliferation rate, cell viability and undifferentiated state maintenance rate of human iPS cells in Example 3.
Fig. 3 shows the influence of the addition concentration of poloxamer (Kolliphor P407) on the cell proliferation rate of human iPS cells in Example 4.
Fig. 4 shows the influence of the addition concentration of poly(vinyl alcohol) (PVA) on the cell proliferation rate of human iPS cells in Example 5.
Fig. 5 shows the influence of the addition concentration of polyoxyethylene sorbitan monolaurate (Kolliphor PS20) on the cell proliferation rate of human iPS cells in Example 6.
Fig. 6 shows the effect of poloxamer (Kolliphor P188 BIO) on the precipitation of insulin by stirring in Example 7. The bar in the Figure indicates 100 µm.
Fig. 7 shows the effect of poloxamer (Kolliphor P188 BIO) and poly(vinyl alcohol) (PVA) on the precipitation of insulin by circulation in Example 8. The bar in the upper panel of the Figure indicates 500 µm. The lower panel is an enlarged upper panel and bar in of the Figure indicates 100 µm.
Fig. 8 shows the effect of poloxamer (Kolliphor P188 BIO) and poly(vinyl alcohol) (PVA) on the precipitation of insulin by stirring in Example 9. The bar in the Figure indicates 500 µm.
Fig. 9 shows the influence of the addition of poloxamer (Kolliphor P188 BIO) on differentiation induction of human iPS cell in Example 10.

### [Description of Embodiments]

The present invention provides an additive for suspension culture of animal cells that is added to a medium containing insulin and the like for suspension culture of animal cells (hereinafter to be also referred to as "the additive of the present invention" in the present specification).

The additive of the present invention contains a water-soluble polymer.

In the present invention, the "water-soluble polymer" refers to a polymer that has a hydrophilic group in a molecule and is miscible or soluble in water. In the present invention, a polymer having solubility of not less than 5 wt% in water at 25°C is preferably used.

While the water-soluble polymer is not particularly limited, a water-miscible or water-soluble polymer having a weight average molecular weight of about 1,000 - 100,000, as measured by size-exclusion chromatography, is generally used.

Examples of the water-soluble polymer include carboxyvinyl polymer; poly(vinyl alcohol); polyvinylpyrrolidone; polyoxyethylene type non-ionic surfactants such as polyoxyethylene alkyl ether, polyoxyethylene alkylphenylether, polyoxyethylene fatty acid ester, polyoxyethylene polyhydric alcohol fatty acid partial ester (polyoxyethylene glycerol fatty acid partial ester, polyoxyethylene sorbitol fatty acid partial ester, polyoxyethylene sorbitan fatty acid partial ester etc.), polyoxyethylene hydrogenated castor oil, polyoxyethylene alkylamine and the like; polyoxyethylene polyoxypropylene type non-ionic surfactants such as polyoxyethylene polyoxypropylene random copolymer, polyoxyethylene polyoxypropylene block copolymer (poloxamer), polyoxyethylene polyoxypropylene alkylether and the like; polyglycerol type non-ionic surfactants such as polyglycerol fatty acid ester and the like; and the like.

For the purpose of the present invention, a non-ionic water-soluble polymer having surface activity is preferably used as a water-soluble polymer, and preferable examples of the water-soluble polymer include poly(vinyl alcohol), polyoxyethylene type non-ionic surfactant, polyoxyethylene polyoxypropylene type non-ionic surfactant and polyglycerol type non-ionic surfactant. Among these, poly(vinyl alcohol), polyoxyethylene polyhydric alcohol fatty acid partial ester and polyoxyethylene polyoxypropylene block copolymer (poloxamer) are more preferably used, poly(vinyl alcohol), polyoxyethylene sorbitan mono-fatty acid ester (polyoxyethylene sorbitan monolaurate, polyoxyethylene sorbitan monopalmitate, polyoxyethylene sorbitan monostearate, polyoxyethylene sorbitan monooleate etc.), and polyoxyethylene polyoxypropylene block copolymer (poloxamer) are particularly preferably used.

For the additive of the present invention, one kind of the water-soluble polymer may be selected and used alone, or two or more kinds thereof can also be selected and used in combination.

The content of the water-soluble polymer in the additive of the present invention is set so that the content of the water-soluble polymer in the medium composition when added to the medium will fall within the range of the below-mentioned content.

In the present invention, the above-mentioned water-soluble polymer may be used as it is as the additive of the present invention, or may be dissolved or dispersed in a solvent such as water, polyhydric alcohol, or the like and used as a liquid form such as aqueous solution, dispersion or the like, or may be mixed with an additive generally used for formulation such as excipient, binder and the like, then milled, granulated, tableted or the like, and used as an additive in a solid form such as powder, granule, tablet or the like.

In addition, the above-mentioned water-soluble polymer may be mixed with a part of the medium components described below such as carbohydrate, inorganic salt and the like and prepared as the additive of the present invention.

From the viewpoint that the addition to a medium for suspension culture of animal cells is convenient and blending with a medium is easy, the additive of the present invention is preferably provided in the form of liquid, powder, granule, tablet or the like.

The additive of the present invention is preferably prepared through a sterilization treatment. The method of the sterilization treatment is not particularly limited, and examples thereof include autoclave sterilization at 121°C for 20 min, radiation sterilization, ethylene oxide gas sterilization, filter filtration sterilization, and the like. The method can be appropriately selected according to the form and the like of the additive of the present invention.

The additive of the present invention is added to the components of the below-mentioned medium for suspension culture of animal cells, and used for preparation of a medium for suspension culture of animal cells, or used by adding to the below-mentioned medium for suspension culture of animal cells.

Precipitation of a medium component such as insulin or the like, which is caused by physical stimulation such as stirring, shaking, circulation, gas bubbling or the like can be suppressed well by adding the additive of the present invention to a medium containing insulin and the like for suspension culture of animal cells, particularly when the aforementioned medium for suspension culture of animal cells is a serum-free medium or a low albumin medium. Thus, cell aggregates with controlled size can be efficiently formed, and the culture efficiency of animal cells and the quality of cultured cells can be improved.

Precipitation of medium components during maintenance culture and differentiation induction can be suppressed by adding the additive of the present invention to a maintenance medium or a differentiation induction medium for undifferentiated cells such as stem cell, and the like, and both the undifferentiated state maintenance rate during maintenance culture and the differentiation rate during differentiation induction can be improved.

The present invention also provides a medium for suspension culture of animal cells (hereinafter to be also referred to as "the medium of the present invention" in the present specification).

As the animal cell here, mammal-derived normal cell, stem cell and progenitor cell can be mentioned.

As the mammal-derived normal cell, germ cells such as spermatozoon, ovum and the like, and somatic cell constituting the living body can be mentioned.

Examples of the somatic cell constituting the living body include, but are not limited to, fibroblast, bone marrow cell, B lymphocyte, T lymphocyte, neutrophil, erythrocyte, platelet, macrophage, monocyte, osteocyte, bone marrow cell, pericyte, dendritic cell, adipocyte, mesenchymal cell, epithelial cell, epidermal cell (e.g., keratinocyte, corneocyte etc.), endothelial cell, vascular endothelial cell, hepatocyte, chondrocyte, cumulus cell, nerve cell, glial cell, oligodendrocyte, micro glia, astrocyte, heart cell, esophageal cell, muscle cells (e.g., smooth muscle cell, skeleton muscle cell), pancreatic beta cell, melanocyte and mononuclear cell and the like.

The somatic cell includes, for example, cells collected from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, blood vessel tissue, blood (including cord blood), bone marrow, heart, eye, brain, neural tissue and the like.

The stem cell refers to a cell that has self-renewal ability and the ability to differentiate into another type of cell and can proliferate infinitely.

Examples include adult stem cell such as hematopoietic stem cell, satellite cell, neural stem cell, mesenchymal stem cell, mammary gland stem cell, olfactory mucosa stem cell, neural crest stem cell, hepatic stem cell, pancreatic stem cell, muscle stem cell, germline stem cell, intestinal stem cell, hair follicle stem cell and the like; pluripotent stem cell such as embryonic stem cell (ES cell), embryonic tumor cell, embryonic germ cell, induced pluripotent stem cell (iPS cell) and the like; cancer stem cell and the like.

Progenitor cell is a cell in the process of differentiating from the aforementioned stem cell into a specific somatic cell or germ cell, and satellite cell, pancreatic progenitor cell, vascular progenitor cell, endothelial progenitor cell, and hematopoietic progenitor cell (cord blood-derived CD34 positive cell, etc.) can be mentioned.

The medium of the present invention is preferably provided as a medium for suspension culture of stem cells, more preferably a medium for suspension culture of adult stem cells, embryonic stem cells, and induced pluripotent stem cells, further preferably a medium for suspension culture of embryonic stem cells and induced pluripotent stem cells.

The medium of the present invention contains a water-soluble polymer together with the medium components generally used for the above-mentioned suspension culture of animal cells.

The water-soluble polymer contained in the medium of the present invention is as described above for the additive of the present invention, and the medium of the present invention can contain only one kind of the water-soluble polymer or two or more kinds of the water-soluble polymers in combination.

In the present invention, the water-soluble polymer may be contained in the form prepared as the above-mentioned additive of the present invention and together with the aforementioned medium component, or may be directly added to the medium component.

The content of the water-soluble polymer in the medium of the present invention is generally 0.1 µg/mL - 10 mg/mL, preferably 1 µg/mL - 5 mg/mL, more preferably 10 µg/mL - 5 mg/mL, further preferably 10 µg/mL - 1 mg/mL, as the final concentration during culturing.

Examples of the medium component that can be contained in the medium of the present invention include medium components generally used for culturing animal cells. For example, sugar such as glucose, fructose, sucrose, maltose and the like; amino acid such as asparagine, aspartic acid, glutamine, glutamic acid and the like; protein such as albumin, transferrin and the like; peptide such as glycylglycylglycine, soybean peptide and the like; serum; vitamin such as vitamin A, vitamin B group (thiamine, riboflavin, pyridoxine, cyanocobalamin, biotin, folic acid, pantothenic acid, nicotinamide etc.), vitamin C, vitamin E and the like; fatty acid such as oleic acid, arachidonic acid, linoleic acid and the like; lipid such as cholesterol and the like; inorganic salt such as sodium chloride, potassium chloride, calcium chloride, magnesium sulfate, sodium dihydrogen phosphate and the like; trace element such as zinc, copper, selenium and the like; buffering agent such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine) and the like; antibiotic such as amphotericin B, kanamycin, gentamicin, streptomycin, penicillin and the like; cell adhesion factor and extracellular matrix component such as type I collagen, type II collagen, fibronectin, laminin, poly-L-lysine, poly-D-lysine and the like; cytokine and growth factor such as interleukin, fibroblast growth factor (FGF), hepatocyte growth factor (HGF), transforming growth factor (TGF)-α, transforming growth factor (TGF)-β, vascular endothelial growth factor (VEGF), activin A and the like; hormone such as dexamethasone, hydrocortisone, estradiol, progesterone, glucagon, insulin and the like, and the like can be mentioned. An appropriate component can be selected and used according to the type of the animal cells to be cultured.

When the animal cell is an undifferentiated cell such as stem cell and the like, a component that suppresses the differentiation of stem cells and the like can be added to the maintenance medium for maintaining the stem cells and the like in an undifferentiated state.

A component that induces or promotes differentiation of stem cell and the like can be added to a differentiation induction medium that induces the differentiation of stem cells and the like.

Examples of the component that suppresses differentiation of stem cell and the like include leukemia inhibitory factor (LIF), which is an inhibitor of differentiation of embryonic stem cells, fibroblast growth factor (FGF), transforming growth factor (TGF)-β, bone morphogenic factor that suppresses differentiation of neural stem cells (bone morphogenetic protein; BMP), Notch protein, Polycomb complex that suppresses differentiation of embryonic stem cells and iPS cells, and the like.

Examples of the component that induces or promotes differentiation of stem cells and the like include activin A that leads embryonic stem cells to endoderm cells, retinoic acid, bone morphogenetic factor (BMP) inhibitor (noggin, etc.) that induces differentiation of iPS cells into neuroectoderm, transforming growth factor (TGF)-β, extracellular secretory glycoprotein (WNT) that induces differentiation of iPS cells into mesoderm, activin that induces differentiation of iPS cells into mesoderm and endoderm, glycogen synthase kinase 3 (GSK3) inhibitor, and the like.

When ectodermal cell, mesodermal cell, and endodermal cell are differentiated into the cells of organ or tissue after early differentiation, a necessary growth factor, a nutrition factor and the like can be added according to the organ and tissue into which they are induced to differentiate. For example, brain-derived neurotrophic factor (BDNF), glial cell line-derived neurotrophic factor (GDNF), fibroblast growth factor (FGF), bone morphogenic factor (BMP), hepatocyte growth factor (HGF) and the like are used.

Since serum may contain unidentified factor, prion, virus and the like, it is preferable that the medium of the present invention be free of a serum as the medium component. In addition, when the medium of the present invention is prepared as a medium for culturing human cells, it is preferable that the medium be free of a component derived from an animal other than human.

In the present invention, moreover, the water-soluble polymer may be contained in a medium widely used for suspension culture of animal cells such as the above-mentioned mammal-derived normal cell, stem cell, progenitor cell and the like, and the medium may be used as the medium of the present invention.

Examples of the medium used for culturing mammalian cell-derived normal cells include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the medium used for culturing stem cells include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek Ltd.), NutriStem (trade mark) XF/FF Culture medium (Stemgent), AF NutriStem (registered trade mark) hESC XF medium (Biological Industries Israel Beit-Haemek Ltd.), S-medium (DS Pharma Biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific) and the like.

As the medium to be used for culturing progenitor cells, HPGM (trade mark) (Cambrex Corporation), QBSF-60 (Quality Biological, Inc.) and the like can be mentioned.

In the present invention, moreover, a water-soluble polymer may be added to the differentiation induction medium for stem cell and the like.

Examples of the differentiation induction medium for stem cell and the like include TeSR-E6 medium (STEMCELL Technologies), TeSR-E7 medium (STEMCELL Technologies), Essential 6 (Thermo Fisher Scientific) and the like.

For the purpose of the present invention, a feeder-free medium for culturing animal cells is preferably used, and a serum-free medium or low albumin medium is more preferably used. In addition, a medium for culturing human cells preferably does not contain a component derived from an animal other than human (xeno-free medium).

The medium of the present invention is preferably a serum-free medium or low albumin medium containing insulin since the effects of the present invention are more remarkably achieved.

Furthermore, from the aspect that it is used for suspension culture of animal cells, the medium of the present invention is preferably in the form of a liquid such as solution, dispersion or the like.

The medium of the present invention can be prepared by adding a component appropriately selected from the above-mentioned medium components together with the water-soluble polymer to a solvent such as water and the like according to a known composition, and dissolving or dispersing them.

The medium of the present invention can also be prepared by adding the water-soluble polymer to the above-mentioned medium for culturing animal cells which is provided by each company or institution, and dissolving or dispersing them.

Furthermore, the medium of the present invention can also be prepared in a state concentrated relative to the concentration at the time of use, or as a freeze-dried powder, and used by diluting with a solvent such as water and the like, or by dissolving in a solvent such as water and the like.

The medium of the present invention is preferably prepared by applying a sterilization treatment as mentioned above.

Suspension culture of animal cells using the medium of the present invention can favorably suppress precipitation of a medium component such as insulin or the like, which is produced by physical stimulation such as stirring, shaking, circulation, gas bubbling or the like, cell aggregates with controlled size can be efficiently formed, and the culture efficiency of animal cells and the quality of cultured cells can be improved.

The medium of the present invention can be preferably used as a medium for maintenance culture or a medium for differentiation induction of undifferentiated cells such as stem cell and the like, can suppress precipitation of a medium component during maintenance culture or differentiation induction of undifferentiated cells such as stem cell and the like, and can improve both the undifferentiated state maintenance rate during maintenance culture and the differentiation rate during differentiation induction.

Furthermore, the present invention provides a method for suspension culture of animal cells (hereinafter to be also referred to as "the culture method of the present invention" in the present specification).

The culture method of the present invention includes suspension culturing animal cells in a medium for suspension culture of animal cells containing a water-soluble polymer.

The "medium for suspension culture of animal cells containing water-soluble polymer" is as described above. The water-soluble polymer which is contained in the medium for suspension culture of animal cells in the present invention may be one prepared and added as the above-mentioned additive of the present invention, or the water-soluble polymer itself may be directly added.

In the present invention, the water-soluble polymer is added to the medium such that the final concentration at the time of culture would be generally 0.1 µg/mL - 10 mg/mL, preferably 1 µg/mL - 5 mg/mL, more preferably 10 µg/mL - 5 mg/mL, further preferably 10 µg/mL - 1 mg/mL.

In the culture method of the present invention, the suspension culture of animal cells can be performed according to a general method for suspension culture. That is, using a culture device or culture apparatus such as a cell culture plate, a cell culture flask, a bioreactor or the like as appropriate according to the culture scale, animal cells are seeded in the above-mentioned medium of the present invention or a medium for suspension culture of animal cells added with the additive of the present invention and cultured at generally 25°C - 39°C, preferably 33°C - 39°C, in the presence of generally 4% by volume - 10% by volume, preferably 4% by volume - 6% by volume, of carbon dioxide, and in the presence of generally 1% by volume - 25% by volume, preferably 4% by volume - 20% by volume, of oxygen for generally 1 day - 30 days, preferably 2 days - 14 days. The medium is exchanged every 2 - 3 days.

To exchange the medium, the animal cells and the medium may be separated by centrifugation or filtration, and then a new medium may be added to the animal cells. Alternatively, animal cells may be appropriately concentrated by centrifugation or filtration, and then a new medium may be added to the cell concentrate.

The acceleration of gravity (G) during the above-mentioned centrifugation is generally 50G - 1,000G, preferably 100G - 500G, and the size of the fine pores in the filter to be used for filtration is generally 10 µm - 200 µm.

The culture method of the present invention can be performed by stirring, shaking, circulation, gas bubbling and the like.

Stirring can be performed using a bioreactor, culture tank with impeller and the like.

Stirring is performed at a stirring rate of generally 10 rpm - 2,000 rpm, preferably 40 rpm - 1,000 rpm.

Shaking can be performed using a shaker or a shaking incubator.

Shaking is generally performed at a shaking rate of 10 rpm - 500 rpm, preferably 50 rpm - 250 rpm.

Circulation can be performed using a peristaltic pump, tubing pump and the like. As the tube for circulation, tube for peristaltic pump, tube for tubing pump and the like made of silicone, Neoprene (chloroprene rubber), Marprene (polypropylene-ethylenepropylene rubber), and the like.

Circulation is generally performed at a flow rate of 10 µL/min - 1000 mL/min, preferably 1 mL/min - 100 mL/min.

Gas bubbling can be performed using various spargers such as micro sparger, filter sparger and the like.

Gas bubbling can be generally performed at a gas flow rate of 1 mL/min - 1000 mL/min, preferably 50 mL/min - 200 mL/min.

To efficiently obtain a cell aggregate having a controlled size, it is preferable to suspension culture the animal cells with stirring or shaking.

The cultured animal cells can be recovered by centrifugation or filtration using a filter.

Centrifugation is performed at 50 G - 1,000 G, preferably 100 G - 500 G, for about 1 min - 10 min.

Filtration can be performed using a filter with fine pores of about 10 µm - 200 µm.

The cultured animal cells are preferably preserved using a freezing medium containing a cryoprotective agent such as STEM-CELLBANKER (Nippon Zenyaku Kogyo Co., Ltd.) and the like in liquid nitrogen.

The culture method of the present invention can favorably suppress precipitation of a medium component such as insulin or the like, which is produced by physical stimulation when suspension culture of animal cells is performed by stirring, shaking, circulation, gas bubbling or the like, and suspension culture can be performed forming cell aggregates with a controlled size. As a result, the culture efficiency of animal cells and the quality of cultured cells can be improved.

The culture method of the present invention can be preferably used for both the maintenance culture and differentiation induction of undifferentiated cells such as stem cell and the like, can suppress precipitation of a medium component during maintenance culture or differentiation induction of undifferentiated cells such as stem cell and the like, and can improve both the undifferentiated state maintenance rate during maintenance culture and the differentiation rate during differentiation induction.

### [Example]

The present invention is explained in more detail in the following by referring to Examples.

In the following Examples, using the medium for stem cell culture described below, the following water-soluble polymer, and undifferentiated human iPS cell (hiPSC) as the stem cell, suspension culture was performed by stirring as shown below.
(1) As the media for culturing stem cells, Essential 8 medium (Thermo Fisher Scientific, A1517001), StemFit (registered trade mark) AK03N medium (Ajinomoto Co., Inc.), and the below-mentioned differentiation induction medium were used.
(2) As the water-soluble polymer, poly(vinyl alcohol) (PVA) (The Nippon Synthetic Chemistry Co., Ltd., EG-03P), poloxamer (polyoxyethylene (160) polyoxypropylene (27) block copolymer) (Kolliphor (registered trade mark) P188 BIO) (BASF), poloxamer (polyoxyethylene (202) polyoxypropylene (56) block copolymer) (Kolliphor (registered trade mark) P407) (BASF), poloxamer (polyoxyethylene (20) polyoxypropylene (20) block copolymer) (Kollisolv P124) (BASF), polyoxyethylene (20) sorbitan monolaurate (Kolliphor PS 20 (BASF), polyoxyethylene (20) sorbitan monopalmitate (Kolliphor PS 40) (BASF), and polyoxyethylene (20) sorbitan monooleate (Kolliphor PS 80 (BASF) were used.
(3) Evaluation of precipitation of insulin in medium by stirring
   A single-use bioreactor (5 mL volume, ABLE Corporation, S-1467) was used as a stirring culture apparatus. A medium (5 mL) was added to the aforementioned culture apparatus, and stirred under the conditions of 37°C, 5% by volume carbon dioxide and 20% by volume oxygen at 120 rpm for 24 hr. Thereafter, the medium was transferred to a 6-well cell culture plate, observed under an inverted microscope ("CKX41", Olympus Corporation, magnification=x40), and photographed.
(4) Suspension cell culture by stirring of undifferentiated human iPS cell (hiPSC)

As an undifferentiated human iPS cell (hiPSC), 1210B2 line of hiPS cells (see Nakagawa, M. et al., Sci. Rep. 4, 3594, 2014) were used.

Suspension cell culture by stirring was performed using single-use bioreactor (5 mL volume, ABLE Corporation, S-1467) as the culture apparatus.

A medium (5 mL) containing 10 µM Rho-associated kinase inhibitor (Y-27632) (Fujifilm Wako Pure Chemical Corporation, 034-24024) was added to the above-mentioned bioreactor, single-celled hiPSCs were added, and stirring culture was performed under conditions of 37°C, 5% by volume carbon dioxide and 20% by volume oxygen at 80 rpm.

The medium was exchanged from day 2 and thereafter. The medium was exchanged by drawing the medium supernatant in the amount indicated in each Example, centrifuging same at 200G for 5 min, removing the supernatant, adding the same amount of a fresh medium, suspending pellets and adding the suspension to the bioreactor.

In each of the following Examples, the measurement of the number of cell aggregates and their major axis, the measurement of cell number and survival rate, the measurement of the undifferentiated state maintenance rate in the cultured stem cells, and the measurement of the differentiation rate into embryonic endoderm cell were performed as described below.

### (1) Measurement of the number of cell aggregates and their major axis

The medium supernatant containing the cell aggregates (500 µl) was collected on a 24-well plate. The cell aggregates were dispersed by shaking, and the entire well was photographed with a BZ-X fluorescence microscope (Keyence). By macrocell counting on the obtained image, the number and average major axis of the cell aggregates were determined.

### (2) Measurement of cell number and survival rate

The total amount of the medium supernatant containing the cell aggregates was recovered, and centrifuged at 500 G for 5 min. After removing the supernatant, tapping was performed 10 times, 1 mL of cell separation/dispersion solution (Accumax (Millipore, SCR006)) was added, and the cell aggregate pellet was suspended. After incubating for 5 min at room temperature, the cell aggregate was resuspended by pipetting. After incubating again for 5 min at room temperature, the cell aggregate was single-celled by pipetting. The medium (4 mL) was added and the mixture was centrifuged at 500 G for 5 min. After removing the supernatant, the pellets were disrupted by tapping 10 times. The cells were resuspended by adding 1 mL of a medium containing Rho-associated kinase inhibitor (Y-27632) and pipetting. The suspension was passed through a 40 µm cell strainer (BD Falcon (Corning Incorporated), 2-1919-02), and the cell strainer was prewashed with 1 mL of a medium containing a Rho-associated kinase inhibitor (Y-27632). The number of cells and the survival rate were measured by analyzing the collected cell suspension with a cell viability autoanalyzer Vi-CELL XR (Beckman Coulter).

The number of viable cells in the recovered cells was divided by the total number of recovered cells to determine the cell viability, and the number of viable cells was divided by the number of seeded cells to determine the cell proliferation rate (fold).

### (3) Measurement of undifferentiated state maintenance rate

The cells single-celled after culturing were immobilized with a cell immobilization/cell permeabilization solution (BD Cytofix/Cytoperm (trade mark) Kit (BD Biosciences, 554714)). Specifically, 200 µL of Cytofix/Cytoperm was added, and the hiPSCs were allowed to stand on ice for 20 min to fix them.

Then, 1 mL of BD Perm/Wash buffer (trade mark) (BD Biosciences, 554723) was added, and the mixture was centrifuged at 5,000 rpm for 2 min to remove the supernatant. Then, it was suspended in an adequate amount of BD Perm/Wash buffer (trade mark), a sample for double staining, a sample for single staining, a sample for isotype control, and a sample for non-staining were each separately dispensed into a centrifuge tube, centrifuged at 5,000 rpm for 2 min, and the supernatant was removed.

Double staining and single staining were performed by adding 100 µL of a solution obtained by adding one or both of 1:5 (5-fold) diluted Alexa Fluor (registered trade mark) 488 mouse anti-oct3/4 (Becton Dickinson, 560253) and 1:10 (10-fold) diluted Alexa Fluor (registered trade mark) 647 mouse anti-SSEA-4 (Becton Dickinson, 560796) to BD Perm/Wash buffer (trade mark), and incubating at room temperature under shading for 20 min.

To the isotype control sample was added 100 µL of BD Perm/Wash buffer (trade mark) added with 1:20 (20-fold) diluted Alexa Fluor (registered trade mark) 488 Mouse IgG1 κ Isotype Control (Becton Dickinson, 557721) or 1:20 (20-fold) diluted Alexa Fluor (registered trade mark) 647 Mouse IgG3, κ Isotype Control (Becton Dickinson, 560803), and the mixture was incubated similarly at room temperature under shading for 20 min.

After each of the above-mentioned reactions, 500 µL of BD Perm/Wash buffer (trade mark) was added, and the mixture was centrifuged at 5,000 rpm for 2 min to remove the supernatant. To each sample was added 1 mL of Focusing fluid (Thermo Fisher Scientific, 4488621), the mixture was centrifuged again at 5,000 rpm for 2 min and suspended in 200 µL of Focusing fluid (Thermo Fisher Scientific, 4488621). The prepared samples were analyzed by Attune NxT Flow Cytometer (Thermo Fisher Scientific). Alexa Fluor (registered trade mark) 488 was detected with BL1, and Alexa Fluor (registered trade mark) 647 was detected with RL1.

The undifferentiated state maintenance rate of cell can be shown by an Oct3/4/SSEA4 positive rate of the cultured cells.

### (4) Measurement of differentiation rate of hiPSC into embryonic endoderm cell

Cells single-celled after culturing were centrifuged at 5,000 rpm for 2 min. The cell pellets were suspended in 100 µL of BD Perm/Wash buffer (trade mark) (BD Biosciences, 554723), 1 µL of BD Pharmingen (trade mark) APC Mouse Anti-Human CD184 (BD Biosciences, 560936) or 1 µL of BD Pharmingen (trade mark) APC Mouse IgG2a, κ Isotype Control (BD Biosciences, 555576) was added, and the mixture was stained by incubation at room temperature under shading for 20 min. The stained cells were washed once with 1000 µL of FACS buffer, centrifuged again to give pellets which were immobilized with a cell immobilization/cell permeabilization solution (BD Cytofix/Cytoperm (trade mark) Kit (BD Biosciences, 554714)). Specifically, 200 µL of Cytofix/Cytoperm was added, and the cells were allowed to stand on ice for 20 min to fix them.

Then, 1 mL of BD Perm/Wash buffer was added, and the mixture was centrifuged at 5,000 rpm for 2 min to remove the supernatant, and suspended in 200 µL of BD Perm/Wash buffer (trade mark). 1 µL of BD Pharmingen (trade mark) PE Mouse anti-Human Sox17 (BD Biosciences, 561591) or BD Pharmingen (trade mark) PE Mouse IgG1,κ Isotype Control (BD Biosciences, 400139) was added, and the mixture was reacted by incubation at room temperature under shading for 20 min.

After the above-mentioned reactions, 500 µL of BD Perm/Wash buffer (trade mark) was added, and the mixture was centrifuged at 5,000 rpm for 2 min to remove the supernatant. To each sample was added 1 mL of Focusing fluid (Thermo Fisher Scientific, 4488621), the mixture was centrifuged again at 5,000 rpm for 2 min and suspended in 200 µL of Focusing fluid (Thermo Fisher Scientific, 4488621). The prepared samples were analyzed by Attune NxT Flow Cytometer (Thermo Fisher Scientific). PE was detected with BL1, and APC was detected with RL1.

The cell differentiation rate can be shown by the CXCR4 or SOX17 positive rate of the cultured cells.

### [Example 1] Study of effect of various water-soluble polymers on insulin precipitation

To Essential 8 medium containing insulin was added each of the above-mentioned water-soluble polymers at 1 mg/mL, and the mixture was stirred in a stirring culture apparatus at 120 rpm for 24 hr as mentioned above. Thereafter, the medium state was observed with an inverted microscope, and the insulin precipitation suppressive effect of the water-soluble polymers was evaluated according to the following evaluation criteria based on the degree of insulin precipitation in the medium.

For comparison, the same treatment was performed without adding a water-soluble polymer, and the degree of insulin precipitation in the medium was evaluated.

The results are shown in Table 1.

### <Evaluation criteria>

Very good (insulin precipitation is completely suppressed); ++ Good (insulin precipitation is almost suppressed); + No suppressive effect (insulin precipitation is observed); -

**[Table 1]**

| water-soluble polymer | evaluation |
|---|---|
| no addition | - |
| poly(vinyl alcohol) | ++ |
| poloxamer (Kolliphor P188 BIO) | ++ |
| poloxamer (Kolliphor P407) | ++ |
| poloxamer (Kollisolv P124) | + |
| polyoxyethylene sorbitan monolaurate | + |
| polyoxyethylene sorbitan monopalmitate | ++ |
| polyoxyethylene sorbitan monooleate | + |

As shown in Table 1, when poly(vinyl alcohol), various poloxamers, and various polyoxyethylene sorbitan mono-fatty acid esters were added, it was found that the precipitation of insulin by stirring was suppressed well.

From the above-mentioned results of Example 1, it was clarified that the precipitation of insulin that occurs when the medium is stirred can be suppressed by adding a water-soluble polymer such as poly(vinyl alcohol) and the like to the insulin-containing medium.

### [Example 2] Study of influence of addition concentration of each of poly(vinyl alcohol) and poloxamer on insulin precipitation suppressive effect

To an Essential 8 medium containing insulin was added 500 ng/mL - 10 mg/mL poly(vinyl alcohol) (PVA) or 100 ng/mL - 1 mg/mL poloxamer (Kolliphor P188 BIO), and the mixture was stirred in a 5 mL bioreactor at 120 rpm for 24 hr as mentioned above. After stirring for 24 hr, the medium was observed with an inverted microscope.

For comparison, the same treatment was performed without adding poly(vinyl alcohol) or poloxamer, and photograph was taken under an inverted microscope.

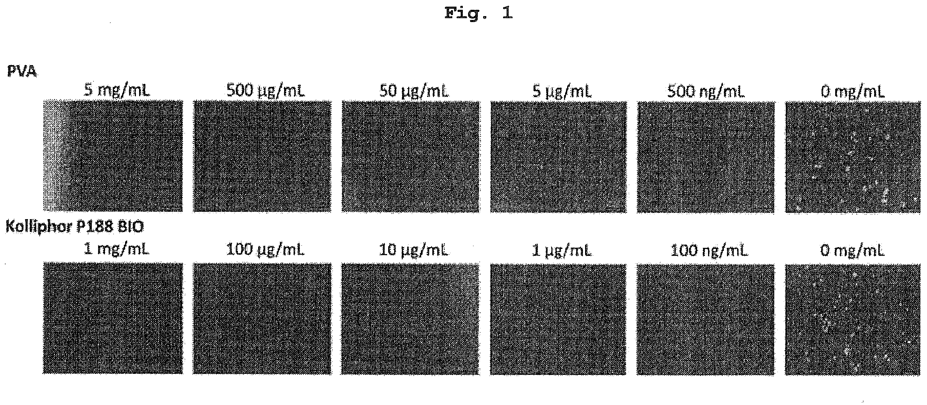

The photograph taken under the microscope is shown in Fig. 1.

As shown in Fig. 1, when the insulin-containing Essential 8 medium was stirred in the bioreactor, precipitation of insulin was observed. In contrast, it was confirmed that when respective concentrations of poly(vinyl alcohol) (PVA) and poloxamer (Kolliphor P188 BIO) were added, insulin precipitation was suppressed at any concentration.

From the above-mentioned results of Example 2, it was clarified that insulin precipitation that occurs by stirring of the medium can be suppressed by adding poly(vinyl alcohol) at not less than 500 ng/mL, and poloxamer (polyoxyethylene (160) polyoxypropylene (27) block copolymer) at not less than 100 ng/mL to the insulin-containing medium.

### [Example 3] Study of effect of poloxamer (Kolliphor P188 BIO) on hiPSC proliferation

To an Essential 8 medium containing insulin was added 0.1 µg/mL - 1 mg/mL poloxamer (Kolliphor P188 BIO), stirring suspension culture of hiPSC was performed for 4 days, and the effect of the poloxamer at each concentration was evaluated.

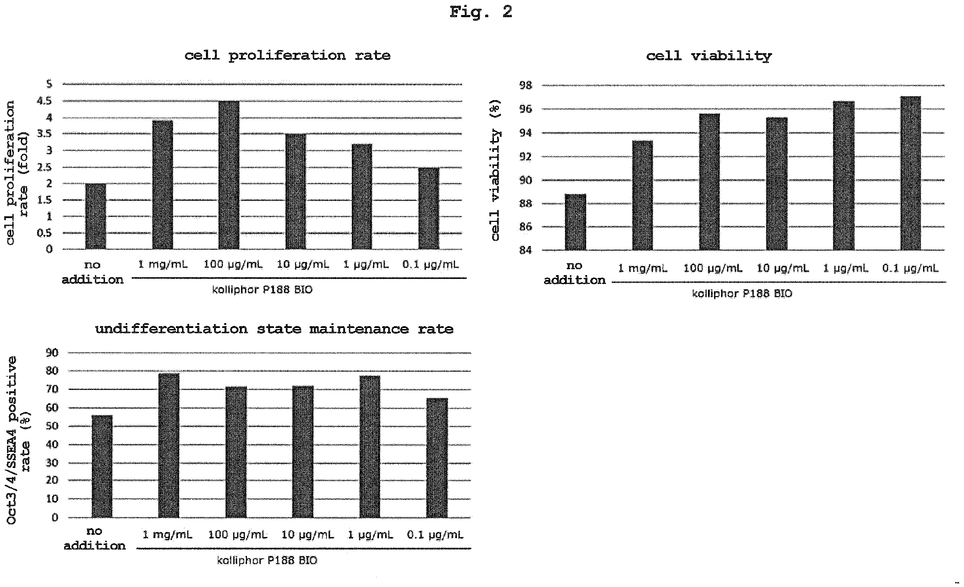

1x10⁶ cells of the 1210B2 line of hiPSC were seeded in a 5 mL bioreactor and cultured with stirring at a stirring rate of 80 rpm as mentioned above. On day 3, 3.5 mL of the medium was exchanged, the cell aggregates were disrupted on day 4, and the cell proliferation rate, survival rate and undifferentiated state maintenance rate were determined. The results are shown in Fig. 2.

As shown in Fig. 2, when stirring suspension culture of hiPSC was performed in a medium added with each concentration of poloxamer (Kolliphor P188 BIO), the cell proliferation rate, survival rate and undifferentiated state maintenance rate were improved.

From the above-mentioned results of Example 3, it was clarified that insulin precipitation in the medium is suppressed, the growth of cell aggregate is promoted, and hiPSC in good condition can be efficiently proliferated by adding poloxamer (polyoxyethylene (160) polyoxypropylene (27) block copolymer) (Kolliphor P188 BIO) to the medium at a concentration of 0.1 µg/mL - 1 mg/mL and performing stirring suspension culture of hiPSC.

### [Example 4] Study of effect of poloxamer (Kolliphor P407) on hiPSC proliferation

To an Essential 8 medium containing insulin was added 1 µg/mL - 1 mg/mL poloxamer (Kolliphor P407), stirring suspension culture of hiPSC was performed for 5 days, and the effect of the poloxamer at each concentration was evaluated.

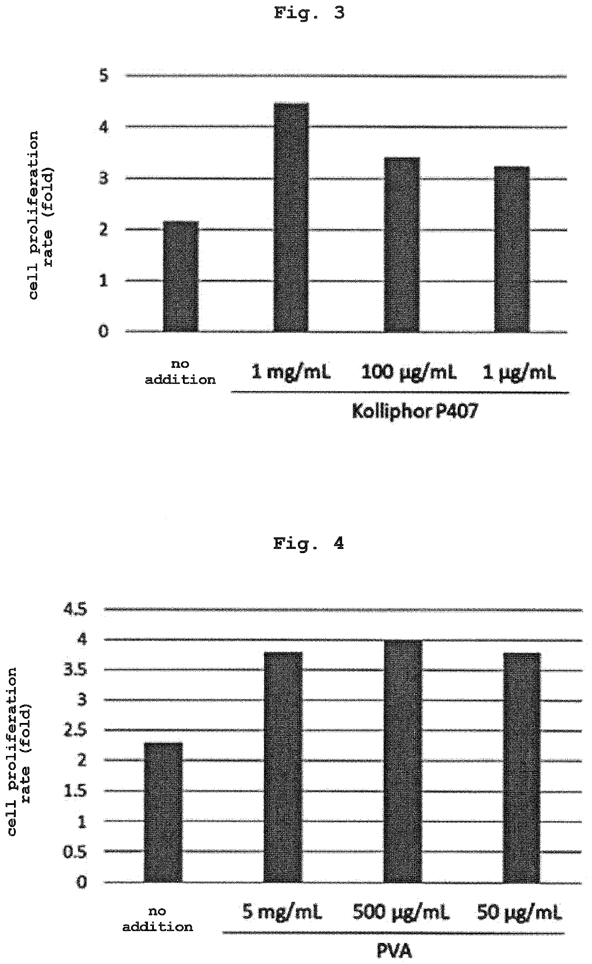

1x10⁶ cells of the 1210B2 line of hiPSC were seeded in a 5 mL bioreactor and cultured with stirring at a stirring rate of 80 rpm as mentioned above. On days 2, 3, 3.5 mL of the medium was exchanged, the cell aggregates were disrupted on day 5, and the cell proliferation rate was determined. The results are shown in Fig. 3.

As shown in Fig. 3, when culture was performed in a medium added with 1 µg/mL - 1 mg/mL poloxamer (Kolliphor P407), the cell proliferation rate was improved.

From the above-mentioned results of Example 4, it was clarified that insulin precipitation in the medium is suppressed, the growth of cell aggregate is promoted, and hiPSC in good condition can be efficiently proliferated by adding poloxamer (polyoxyethylene (202) polyoxypropylene (56) block copolymer) (Kolliphor P407) to the medium at a concentration of 1 µg/mL - 1 mg/mL and performing stirring suspension culture of hiPSC.

### [Example 5] Study of effect of poly(vinyl alcohol) (PVA) on hiPSC proliferation

To an Essential 8 medium containing insulin was added 50 µg/mL - 5 mg/mL poly(vinyl alcohol) (PVA), stirring culture of hiPSC was performed for 4 days, and the effect of poly(vinyl alcohol) at each concentration was evaluated.

1x10⁶ cells of the 1210B2 line of hiPSC were seeded in a 5 mL bioreactor and suspension cultured with stirring at a stirring rate of 80 rpm as mentioned above. On day 2, 3.5 mL of the medium was exchanged, the cell aggregates were disrupted on day 4, and the cell proliferation rate was determined. The results are shown in Fig. 4.

As shown in Fig. 4, when culture was performed in a medium added with 50 µg/mL - 5 mg/mL poly(vinyl alcohol) (PVA), the cell proliferation rate was improved.

From the above-mentioned results of Example 5, it was clarified that insulin precipitation in the medium is suppressed, the growth of cell aggregate is promoted, and hiPSC in good condition can be efficiently proliferated by adding poly(vinyl alcohol) (PVA) to the medium at a concentration of 50 µg/mL - 5 mg/mL and performing stirring suspension culture of hiPSC.

### [Example 6] Study of effect of polyoxyethylene sorbitan monolaurate (Kolliphor PS 20) on hiPSC proliferation

To an Essential 8 medium containing insulin was added 100 ng/mL - 10 µg/mL polyoxyethylene sorbitan monolaurate (Kolliphor PS 20), stirring culture of hiPSC was performed for 4 days, and the effect of polyoxyethylene sorbitan monolaurate at each concentration was evaluated.

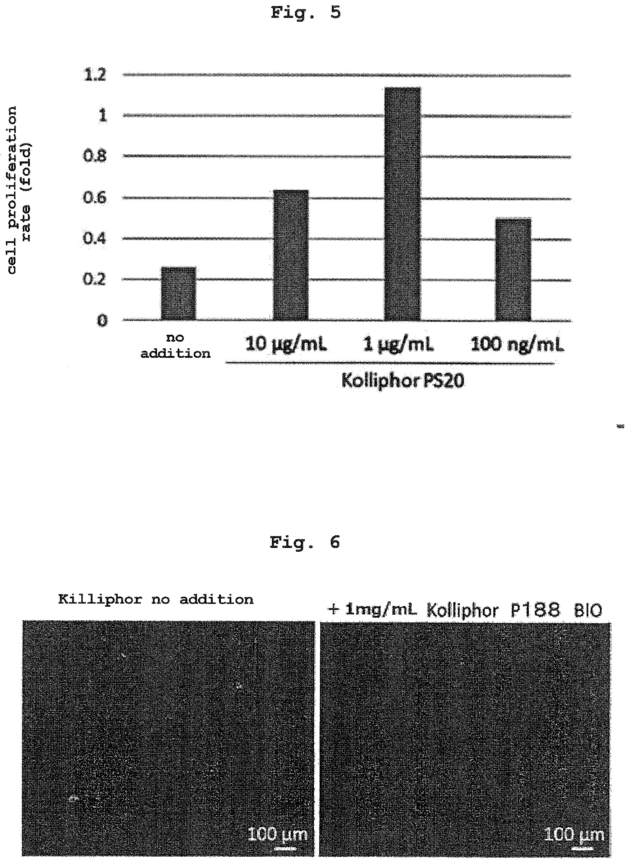

1x10⁶ cells of the 1210B2 line of hiPSC were seeded in a 5 mL bioreactor and suspension cultured with stirring at a stirring rate of 80 rpm as mentioned above. On day 2, 3.5 mL of the medium was exchanged, the cell aggregates were disrupted on day 3, and the cell proliferation rate was determined. The results are shown in Fig. 5.

As shown in Fig. 5, when culture was performed in a medium added with 100 ng/mL - 10 µg/mL polyoxyethylene sorbitan monolaurate (Kolliphor PS 20), the cell proliferation rate was improved.

From the above-mentioned results of Example 6, it was clarified that insulin precipitation in the medium is suppressed, the growth of cell aggregate is promoted, and hiPSC in good condition can be efficiently proliferated by adding polyoxyethylene sorbitan monolaurate (Kolliphor PS 20) to the medium at a concentration of 100 ng/mL - 10 µg/mL and performing stirring culture of hiPSC.

### [Example 7] Study of effect of poloxamer on insulin precipitation by stirring using impeller

Using a 2 L animal cell culture tank (bottom magnetic stirring type) (ABLE Corporation) and a stainless steel (SUS316L) impeller as the stirring culture apparatus, the effect of poroxamer on insulin precipitation by stirring was evaluated.

To the above-mentioned animal cell culture tank were added insulin-containing Essential 8 medium (500 mL), and 1 mg/mL poloxamer (Kolliphor P188 BIO), and the mixture was stirred at 37°C at 150 rpm for 8 hr. Thereafter, the medium was transferred to a 6-well cell culture plate, observed under an inverted microscope ("CKX53", Olympus Corporation, magnification=x100), and photographed.

For comparison, the same treatment was performed without adding poloxamer, and photograph was taken under an inverted microscope.

The photograph taken under the microscope is shown in Fig. 6.

As shown in Fig. 6, it was found that insulin was precipitated by stirring the medium, and addition of poloxamer (Kolliphor P188 BIO) suppressed insulin precipitation.

From the above-mentioned results of Example 7, it was clarified that insulin precipitation that occurs by stirring of the medium by using an impeller can be suppressed by adding poloxamer (polyoxyethylene (160) polyoxypropylene (27) block copolymer) at a concentration of 1 mg/mL to the insulin-containing medium.

### [Example 8] Study of effect of water-soluble polymer on insulin precipitation by circulation

Using a peristaltic pump ("Perista BioMini Pump AC-2120" (ATTO Corporation) as an apparatus for circulating medium, an influence of water-soluble polymer on the insulin precipitation by circulation was evaluated. As a tube for circulation, a silicone tube with inner diameter=3 mm, outer diameter=5 mm was used.

To a 50 mL plastic tube were added insulin-containing Essential 8 medium (45 mL), and poloxamer (Kolliphor P188 BIO) or poly(vinyl alcohol) (PVA) each at 1 mg/mL, and the mixture was circulated using the above-mentioned peristaltic pump at room temperature at flow rate of 5 mL/min for 24 hr. Thereafter, the medium was transferred to a 6-well cell culture plate, observed under an inverted microscope ("CKX41", Olympus Corporation, magnification=x40 and x100), and photographed.

For comparison, the same treatment was performed without adding poloxamer or poly(vinyl alcohol), and photograph was taken under an inverted microscope.

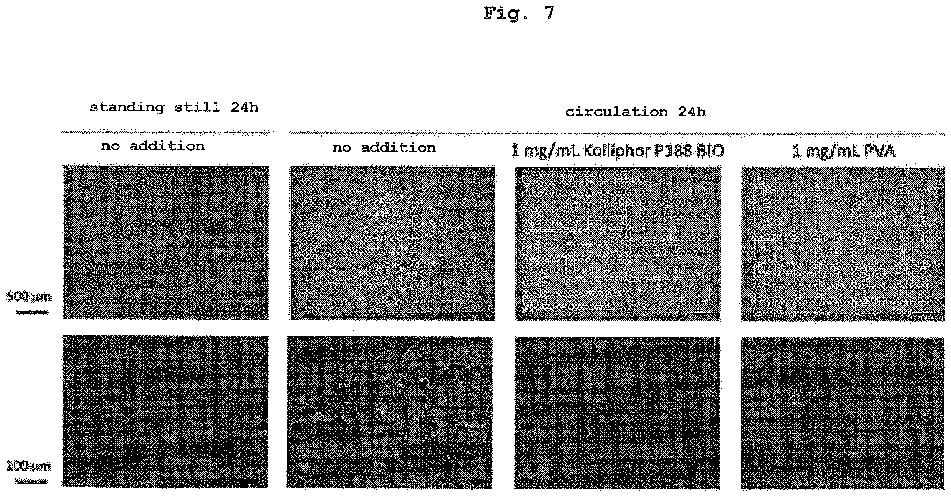

The photograph taken under the microscope is shown in Fig. 7.

As shown in Fig. 7, while insulin precipitation occurs by circulating the medium, it was suppressed by adding poloxamer (Kolliphor P188 BIO) or poly(vinyl alcohol) (PVA) to the medium. From the above-mentioned results of Example 8, it was clarified that insulin precipitation that occurs during circulating the medium can be suppressed by adding poloxamer (polyoxyethylene (160) polyoxypropylene (27) block copolymer) or poly(vinyl alcohol) each at a concentration of 1 mg/mL to the insulin-containing medium.

### [Example 9] Study of effect of water-soluble polymer on insulin precipitation in medium for differentiation induction of stem cell

To a differentiation induction medium containing insulin (RPMI1640 medium added with 4(w/v)% supplement in TeSR-E6 medium (STEMCELL Technologies)) were added 1 mg/mL poly(vinyl alcohol) (PVA) or 1 mg/mL poloxamer (Kolliphor P188 BIO), and the mixture was stirred in a 5 mL bioreactor at 80 rpm for 48 hr as mentioned above. Thereafter, the medium was observed with an inverted microscope CKX41.

For comparison, the same treatment was performed without adding poly(vinyl alcohol) or poloxamer, and observation was performed under an inverted microscope.

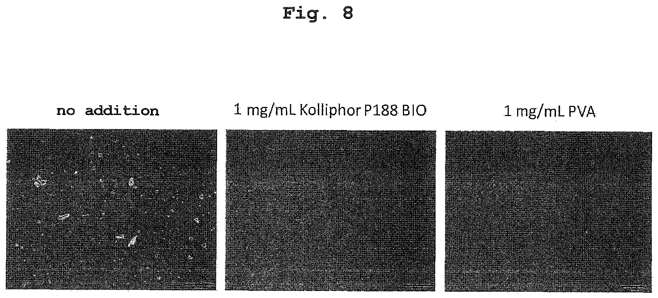

The photograph taken under the microscope as mentioned above is shown in Fig. 8.

As shown in Fig. 8, when the insulin-containing differentiation induction medium was stirred in the bioreactor, precipitation of insulin was observed. In contrast, it was confirmed that when poly(vinyl alcohol) (PVA) and poloxamer (Kolliphor P188 BIO) were added each at 1 mg/mL, insulin precipitation was suppressed.

From the above-mentioned results of Example 9, it was confirmed that insulin precipitation that occurs by stirring the medium can be suppressed by adding poloxamer (polyoxyethylene (160) polyoxypropylene (27) block copolymer) or poly(vinyl alcohol) each at a concentration of 1 mg/mL to the insulin-containing differentiation induction medium.

### [Example 10] Study of effect of poloxamer (Kolliphor P188 BIO) on differentiation induction of hiPSC into embryonic endoderm cell

To a 30 mL bioreactor was added StemFit (registered trade mark) AK03N medium (Ajinomoto Co., Inc.) (30 mL), 6x10⁶ cells of the 1210B2 line of hiPSC were seeded, and stirring suspension culture was performed at 120 rpm for 6 days to form cell aggregates of hiPSC. The cell aggregate (5 mL) was transferred to a 5 mL bioreactor, and stirring suspension culture was performed in a differentiation induction medium (RPMI1640 medium added with 4(w/v)% supplement in TeSR-E6 medium (STEMCELL Technologies)), 2 µM glycogen synthase kinase 3 inhibitor (CHIR99021), 100 ng/mL activin A (Activin A)) added with 0.1 mg/mL poloxamer (Kolliphor P188 BIO) at 80 rpm for 5 days to induce differentiation into embryonic endoderm cell.

For comparison, the same stirring suspension culture was performed without adding poloxamer, whereby differentiation into embryonic endoderm cell was induced.

After differentiation induction, cell aggregates were disrupted, and viable cells number and cell viability were measured as mentioned above. In addition, the proportion of positive cells was quantified for each of the markers CXCR4 and SOX17 of embryonic endoderm cell by the above-mentioned flow cytometry analysis.

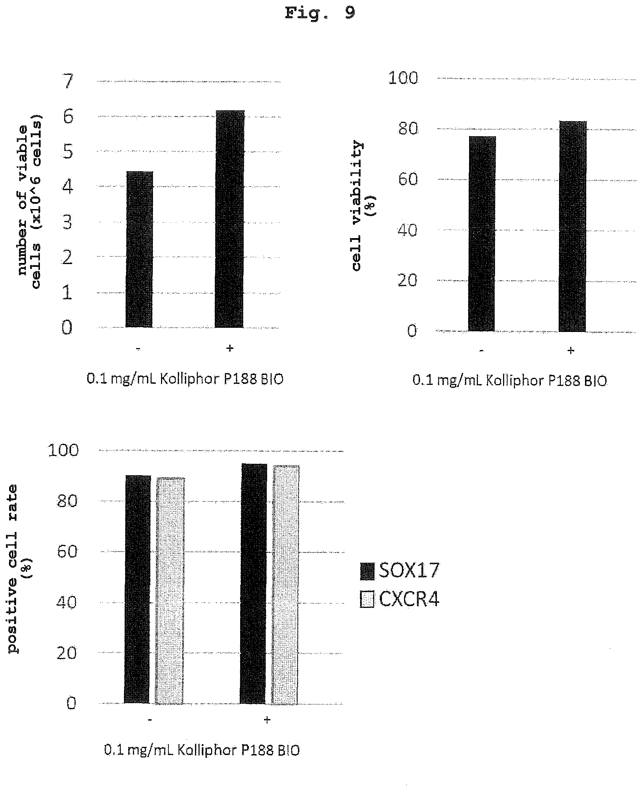

The results are shown in Fig. 9.

As shown in Fig. 9, when stirring suspension culture of hiPSC was performed in a differentiation induction medium added with poloxamer (Kolliphor P188 BIO) to induce differentiation, improvement in the number of viable cells and cell viability was observed.

In addition, the proportion of positive cells increased for each of the embryonic endoderm cell markers CXCR4 and SOX17.

From the above-mentioned results of Example 10, it was confirmed that the cell proliferation rate and survival rate increased by stirring suspension culture of hiPSC in a differentiation induction medium containing poloxamer (polyoxyethylene (160) polyoxypropylene (27) block copolymer), and differentiation into embryonic endoderm cell is also promoted.

### [Industrial Applicability]

As described in detail above, the present invention can provide an additive capable of favorably suppressing precipitation of a medium component such as insulin or the like, which is produced by physical stimulation such as stirring, shaking, circulation, gas bubbling or the like, by adding same to a medium containing insulin and the like for suspension culture of animal cells.

The present invention can also provide a medium containing insulin and the like for suspension culture of animal cells, in which precipitation of medium components such as insulin and the like is suppressed well even when physical stimulation such as stirring, shaking, circulation, gas bubbling or the like is applied, and suspension culture of animal cells can be performed while performing stirring, shaking, circulation, gas bubbling and the like in a medium containing insulin and the like for suspension culture of animal cells.

As a result, the present invention can form cell aggregates with controlled size and perform suspension culture of animal cells, and can improve the culture efficiency of animal cells and the quality of cultured cells.

In particular, precipitation of medium components is suppressed during both maintenance culture of undifferentiated cells such as stem cell and the like in a maintenance medium and differentiation induction thereof in a differentiation induction medium, and both the undifferentiated state maintenance rate during maintenance culture and the differentiation rate during differentiation induction can be improved.

This application is based on a patent application No. 2018-141909 filed in Japan, the contents of which are incorporated in full herein.

## Claims

1. An additive for suspension culture of an animal cell, comprising a water-soluble polymer.

2. The additive according to claim 1, wherein the water-soluble polymer is a non-ionic water-soluble polymer having surface activity.

3. The additive according to claim 2, wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.

4. The additive according to any one of claims 1 to 3, wherein the animal cell is a stem cell.

5. The additive according to claim 4, wherein the stem cell is one kind or two or more kinds selected from the group consisting of an adult stem cell, an embryonic stem cell, and an induced pluripotent stem cell.

6. The additive according to any one of claims 1 to 5, wherein the additive is for suppressing precipitation of a medium component.

7. The additive according to claim 6, wherein the medium component is insulin.

8. A medium for suspension culture of an animal cell, comprising a water-soluble polymer.

9. The medium according to claim 8, wherein the water-soluble polymer is a non-ionic water-soluble polymer having surface activity.

10. The medium according to claim 9, wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.

11. The medium according to any one of claims 8 to 10, wherein the medium is for suspension culture of a stem cell.

12. The medium according to claim 11, wherein the stem cell is one kind or two or more kinds selected from the group consisting of an adult stem cell, an embryonic stem cell and an induced pluripotent stem cell.

13. The medium according to any one of claims 8 to 12, wherein precipitation of a medium component is suppressed.

14. The medium according to claim 13, wherein the medium component is insulin.

15. A method for suspension culture of an animal cell, comprising suspension culturing the animal cell in a medium comprising a water-soluble polymer.

16. The method according to claim 15, wherein the water-soluble polymer is a non-ionic water-soluble polymer having surface activity.

17. The method according to claim 16, wherein the non-ionic water-soluble polymer having surface activity is one kind or two or more kinds selected from the group consisting of poly(vinyl alcohol), a polyoxyethylene polyoxypropylene block copolymer, and a polyoxyethylene sorbitan mono-fatty acid ester.

18. The method according to any one of claims 15 to 17, wherein the animal cell is a stem cell.

19. The method according to claim 18, wherein the stem cell is one kind or two or more kinds selected from the group consisting of an adult stem cell, an embryonic stem cell and an induced pluripotent stem cell.

20. The method according to any one of claims 15 to 19, wherein the suspension culturing comprises stirring, shaking, circulation, or gas bubbling.

21. The method according to any one of claims 15 to 20, wherein the animal cell is suspension cultured in a medium in which precipitation of a medium component is suppressed.

22. The method according to claim 21, wherein the medium component is insulin.

23. The method according to any one of claims 15 to 22, wherein the animal cell is suspension cultured by forming a cell aggregate.
